# EUROPÄISCHE PATENTANMELDUNG

(11) **EP 3 093 335 A1**
(43) Veröffentlichungstag der Anmeldung: **16.11.2016**
(21) Anmeldenummer: 15167538.6
(22) Anmeldetag: 13.05.2015
(51) Int. Cl.: C12M 1/00, C07K 1/36

(54) **PROZESSLEITSYSTEM ZUR REGELUNG UND STEUERUNG EINER MODULAR AUFGEBAUTEN ANLAGE ZUR PRODUKTION VON BIOPHARMAZEUTISCHEN UND BIOLOGISCHEN MAKROMOLEKULAREN PRODUKTEN**

(71) Anmelder: Bayer Technology Services GmbH, 51368 Leverkusen (DE)
(72) Erfinder: Dr. Schwan, Peter, 51373 Leverkusen (DE); Dr. Lobedann, Martin, 51069 Köln (DE); Dr. Bernshausen, Jens, 51375 Leverkusen (DE)
(74) Vertreter: BIP Patents

(57) **Zusammenfassung**

Die Erfindung betrifft eine modular aufgebaute Produktionsanlage zur kontinuierlichen Herstellung und / oder Aufbereitung von biopharmazeutischen Produkten, ein computerimplementiertes Verfahren zur Prozesssteuerung der modular aufgebauten Anlage zur Produktion von biopharmazeutischen und biologischen makromolekularen Produkten , insbesondere von Proteinen, z. B. monoklonale Antikörper, Impfstoffe, Nukleinsäuren wie DNA, RNA und Plasmide sowie deren Derivate kontrolliert.

## Beschreibung

Die Erfindung betrifft eine modular aufgebaute Produktionsanlage zur kontinuierlichen Herstellung und / oder Aufbereitung von biopharmazeutischen Produkten, ein computerimplementiertes Verfahren zur Prozesssteuerung der modular aufgebauten Anlage zur Produktion von biopharmazeutischen und biologischen makromolekularen Produkten , insbesondere von Proteinen, z. B. monoklonale Antikörper, Impfstoffe, Nukleinsäuren wie DNA, RNA und Plasmide sowie deren Derivate kontrolliert. Die stark regulierte pharmazeutische Produktion erfordert einen großen zeitlichen, technischen und personellen Aufwand für die Bereitstellung gereinigter und sterilisierter Bioreaktoren und zur Gewährleistung eines keimfreien Produktes. Um Kreuzkontaminationen bei einem Produktwechsel in einer Multi-Purpose-Anlage oder zwischen zwei Produktchargen sicher zu vermeiden, wird außer der Reinigung eine sehr aufwändige Reinigungsvalidierung benötigt, welche bei einer Prozessadaption ggf. wiederholt werden muss.

Dies gilt sowohl für das Upstream-Processing USP, d.h. die Herstellung biologischer Produkte in einem Bioreaktor als auch für das Downstream-Processing DSP, d.h. die Aufreinigung der Fermentationsprodukte.

Der durch die Bereitstellungsprozeduren bedingte Nutzungsausfall der Reaktoren kann insbesondere bei kurzen Nutzungsperioden und häufigem Produktwechsel in der Größenordnung der Reaktorverfügbarkeit liegen. Betroffen sind im USP der biotechnologischen Produktion z.B. die Prozessschritte der Medienherstellung und Fermentation und im DSP das Solubilisieren, Einfrieren, Auftauen, pH-Adjustieren, Produkttrennung wie z. B. durch Chromatographie, Fällen, oder Kristallisieren, das Umpuffern und die Virusinaktivierung.

Um der Forderung an ein schnelles und flexibles Neubeschicken der Produktionsanlage unter Wahrung maximaler Sauberkeit und Keimfreiheit gerecht zu werden, erfreuen sich auf dem Markt Konzepte für eine kontinuierliche Produktion bevorzugt mit Einwegtechnologie eines ständig wachsenden Interesses.

WO2012/078677 beschreibt ein Verfahren und eine Anlage zur kontinuierlichen Aufbereitung von biopharmazeutischen Produkten durch Chromatographie und deren Integration in einer Produktionsanlage insbesondere in einer Einweg-Anlage. Obwohl WO2012/078677 Ansätze zur kontinuierlichen Produktion von biopharmazeutischen und biologischen Produkten liefert, reicht die offenbarte Lösung in der Praxis nicht aus. Insbesondere beschreibt WO2012/078677 die Nutzung von Behältern (= Bags) zwischen nacheinander geschalteten Units. Obwohl WO2012/078677 offenbart, dass der kontinuierliche Prozess gesteuert ("regulated") werden muss, geben die Autoren keine Angabe darüber, wie diese Steuerung erreicht werden kann. Auf eine Regelung wird auch nicht eingegangen. Die verwendeten Behälter sind lediglich durch ihre Kapazität relativ zur Lot-Größe und ggf. Mischeigenschaft definiert und nicht als Puffervolumina zur Ermöglichung einer kontinuierlichen Prozessregelung beschrieben. Eine Nutzung der Behälters in der Regelung ist daher in WO2012/078677 nicht offenbart und daraus nicht abzuleiten.

WO2014/137903 beschreibt eine Lösung zur integrierten kontinuierlichen Herstellung einer Proteinsubstanz in einer Produktionsanlage, umfassend Säulen zur Durchführung der Produktionsschritte, die in Serie verbunden sind. WO2014/137903 offenbart, dass der Produktstrom in dem kontinuierlichen Prozess idealerweise geregelt ist, so dass soweit wie möglich jeder Schritt oder jede Unit gleichzeitig mit einer ähnlichen Förderrate läuft, um die Produktionszeit zu minimieren. WO2014/137903 offenbart die Nutzung von Behältern zwischen aneinander folgenden Units, die den Produktstrom für eine bestimmte Zeit aufnehmen können. Diese sind aber nicht aufgrund ihrer Regeleigenschaften ausgelegt. Eine Nutzung der Behältervolumina in der Regelung ist daher nicht offenbart und daraus nicht abzuleiten.

Ein Verfahren zur Herstellung von biopharmazeutischen und biologischen Produkten umfasst üblicherweise folgende Produktionsschritte, die üblicherweise wie folgt miteinander verschaltet werden:
A. Upstream
   1. Perfusionskultur
   2. Zellrückhaltesystem,
   alternativ zu Schritt 1 und 2 ist eine Fedbatchkultur.
B. Downstream
   3. Zellabtrennung
   4. Puffer- bzw. Medienaustausch bevorzugt mit Konzentrierung
   5. BioBurden-Reduzierung bevorzugt mit Sterilfilter
   6. Capture Chromatographie
      Üblicherweise werden weitere Schritte zur Reinigung des Produktstroms durchgeführt, insbesondere:
   7. Virusinaktivierung
   8. Neutralisation
   9. Optional eine weitere BioBurden-Reduzierung (mit Sterilfilter)

Angesichts der hohen Qualitätsstandards bei der Herstellung von Biopharmazeutika folgen üblicherweise darüber hinaus weitere Schritte:
10. Chromatographische Zwischen- und Feinreinigung
11. BioBurden Reduzierung z. B. mit Sterilfilter
12. Virenfiltration
13. Pufferaustausch und bevorzugt Konzentrierung
14. Filtration mit Sterilfilter.

Eine Produktionsanlage im Sinne der Erfindung umfasst in Serie miteinander verbundene Units zur Durchführung von mindestens zwei Downstream- und / oder Upstream-Schritten, in denen ein Produktstrom befördert werden kann. Erfindungsgemäß sind die Units zur kontinuierlichen oder semi-kontinuierliche Durchführung eines Schrittes geeignet und können mit einem kontinuierlichen Produktstrom betrieben werden.

Ein kontinuierliches Verfahren im Sinne der Anmeldung ist jeder Prozess, zur Durchführung von mindestens zwei Prozessschritten in Serie, in dem der Ausgangsstrom eines vorgeschalteten Schrittes in einen nachgeschalteten Schritt befördert wird. Der nachgeschaltete Schritt beginnt die Bearbeitung des Produktstroms, bevor der vorgeschaltete Schritt abgeschlossen ist. Üblicherweise wird in einem kontinuierlichen Verfahren immer ein Teil des Produktstroms in der Produktionsanlage befördert und wird als kontinuierlicher Produktstrom bezeichnet. Entsprechend bedeutet eine kontinuierliche Beförderung oder Transfer eines Produktstroms von einer vorgeschalteten Unit in eine nachgeschaltete Unit, dass die nachgeschaltete Unit bereits arbeitet, bevor die vorgeschaltete Unit außer Betrieb genommen wird, d. h. dass zwei nacheinander geschaltete Units den Produktstrom gleichzeitig prozessieren, der sie durchfließt. Üblicherweise ergibt sich bei stetigem und kontinuierlichem Ausgangsstrom einer Unit ein stetiger und kontinuierlicher Ausgangsstrom der darauffolgenden Unit.

Erfordert eine Unit-Operation den Wechsel eines Elements zur Durchführung des Schrittes (auch VTE genannt), so kann die Unit im Sinne der Erfindung nur semi-kontinuierlich betrieben werden. Um den kontinuierlichen Betrieb des Gesamtverfahrens zu ermöglichen, können mehrere VTE parallel oder alternierend in der entsprechenden Unit betrieben werden, so dass ein quasikontinuierlicher Strom gewährleistet wird. Alternativ soll die Produktionsanlage die Teilunterbrechung des Produktstroms während des Wechsels der betroffenen Unit ermöglichen. Ein Hybridverfahren im Sinne der Anmeldung ist eine Mischung aus Batch- und Kontinuierlich gefahrenen Schritten beispielsweise alle Schritte als kontinuierlich gefahrene Schritte außer der Diafiltration, die in Batch-Fahrweise betrieben wird.

Die verschiedenen Units einer solchen Produktionsanlage erfordern typischerweise verschiedene Flussraten. Eine Unit, die maßgeblich eine Flussrate bestimmt, wird in dieser Anmeldung als Master Unit bezeichnet; eine Master Unit umfasst mindestens eine Vorrichtung zur Beförderung des Produktstroms, üblicherweise eine Pumpe oder ein Ventil, bevorzugt eine Pumpe. Auch kann die Produktionsanlage mehrere Master Units umfassen.

Ein kontinuierliches Verfahren zur Produktion von biologischen Produkten erfordert ein Konzept für die Beförderung des Produktstroms von einer Unit zu einer darauffolgenden. Herausfordernd dabei ist die Anpassung der Ein- und Ausgangsströme der vor- und nachgeschalteten Unit aufeinander, wenn die Flussraten nicht genau zu einander passen, z. B. grundsätzlich fluktuieren, im Laufe des kontinuierlichen Betriebs variieren oder einfach anders sind. Diese Variationen werden im Stand der Technik durch einen Behälter zur Aufnahme des Produktstroms am Anfang einer Unit aufgefangen.

Typischerweise umfasst eine Produktionsanlage eine automatisierte Steuerung und Regelung der Units durch ein Leitsystem, vornehmlich ein Prozessleitsystem (PLS). Typischerweise ist das Leitsystem mit einer Bedien- und Beobachtungsstation als Schnittstelle, über die der Nutzer den Prozess steuern und beobachten kann, verbunden.

Innerhalb der Automatisierungslogik der Produktionsanlage umfasst das Leitsystem üblicherweise mindestens einen Regler, typischerweise ausgewählt aus einer Gruppe umfassend Hysterese-, PID (proportional-integral-differential)- und Fuzzy-Regler. Die unterschiedlichen Regelalgorithmen werden im Prozessleitsystem entsprechend des Reglertyps konfiguriert:
i. Zwei- oder Mehrpunktregelung optional mit Hysterese
ii. Regelung mithilfe einer Stellwertzuordnung über einen Polygonzug
iii. Fuzzy-Regelung
iv. PID-Regelung - Angabe von proportional, integral und differential Anteil durch Vorgabe von Verstärkung, Nachhalte- und Vorhaltezeit.

In der einfachsten Form einer Automatisierung der Units werden alle Pumpenmotoren der Produktionsanlage aufeinander angepasst und durch manuelle Stellwertvorgabe gesteuert.
Um mehrere Units koordiniert miteinander zu betreiben, ist eine Abstimmung der Flussrate der Units nötig, da zwei Pumpen bei gleicher Drehzahl niemals mit exakt der gleichen Flussrate fördern. Die Flussratendifferenz über die Zeit führt dazu, dass der Füllstand in den Behältern zu oder abnimmt.

Die Aufgabe besteht daher darin, eine Lösung zur Prozesssteuerung einer Anlage zur kontinuierlichen Produktion von biopharmazeutischen und biologischen makromolekularen Produkten bereit zu stellen, die die Nutzung von unterschiedlichen Flussraten, ggf. eine zeitlich beschränkte (Teil-) Unterbrechung des Produktstroms ermöglicht, ohne unmittelbare Auswirkungen auf die kontinuierliche Fahrweise der benachbarte Units zu haben.

Eine Abstimmung der Flussraten erfolgt erfindungsgemäß über die Regelung einer charakteristischen Zustandsgröße der Puffervolumen der Produktionsanlage. Die erfindungsgemäße Lösung basiert auf der Messung und Regelung von Zustandsgrößen, wie z. B. Füllstand und Druck. Erfindungsgemäß wird die Zustandsgröße eines Puffervolumens, bevorzugt jedes Puffervolumens, durch einen Sensor überwacht. Basierend auf den Sensordaten beeinflusst ein Regelalgorithmus mit Hilfe eines geeigneten Aktors die Zustandsgröße des Puffervolumens in einem geschlossenen Wirkungsablauf.

Bevorzugt sind zur Regelung der Zustandsgröße der Puffervolumeneine Hysterese-Regelung, eine Fuzzy-Regelung oder eine PID-Regelung, besonders bevorzugt eine PID-Regelung. Die Fuzzy-Regelung kann z.B. durch einen Polygonzug definiert werden.

Erfindungsgemäß kann das Puffervolumen in einer Unit durch die Anwendung eines dehnbaren Schlauchs oder eines Behälters generiert werden.

Eine Aufgabe des Leitsystems in der vorliegenden Erfindung ist die Einstellung der Flussraten so vorzunehmen, dass eine kontinuierliche Fahrweise des Gesamtprozesses gewährleistet wird und Einflüsse von Störungen innerhalb einzelner Units über die betreffende Unit hinaus minimiert werden. Eine Fortpflanzung von Flussratenschwankungen über eine Unit hinaus kann somit durch die Implementierung geeigneter Regelalgorithmen minimiert werden. Eine weitere Aufgabe des Leitsystems besteht darin, durch Pausieren einer oder mehrerer Units z. B. zu Wartungszwecken ein Überlaufen oder Leerlaufen der Puffervolumen zu verhindern.

In Sinne der Anmeldung bedeutet Regelung, das Messen der zu beeinflussenden Größe (Regelgröße) und ein kontinuierlicher Vergleich mit dem gewünschten Wert (Soll-Wert). Ein Regler errechnet entsprechend der Abweichung eine Stellgröße, die so auf die Regelgröße einwirkt, dass sie die Abweichung minimiert und die Regelgröße ein gewünschtes Zeitverhalten annimmt. Dies entspricht einem geschlossenen Wirkungsablauf.

Im Vergleich bedeutet Steuerung den Vorgang in einem System, bei dem eine oder mehrere Eingangsgrößen die Ausgangsgrößen aufgrund der dem System eigentümlichen Gesetzmäßigkeiten beeinflussen. Kennzeichen für die Steuerung ist der offene Wirkungsweg oder ein geschlossener Wirkungsweg, bei dem die durch die Eingangsgrößen beeinflussten Ausgangsgrößen nicht fortlaufend und nicht wieder über dieselben Eingangsgrößen auf sich selbst wirken (http://public.beuth-hochschule.de/~fraass/MRTII-Umdrucke.pdf). Dies entspricht einem offenen Wirkungsablauf.
Regelung und Steuerung der Produktionsanlage werden auch mit dem Begriff Prozessleitung der Produktionsanlage durch das Leitsystem zusammengefasst.
In Sinne der Anmeldung bedeutet Zielregelung des Puffervolumens, dass der Aktor den Produktstrom in das Puffervolumen hineinfördert.

In Sinne der Anmeldung bedeutet Quellregelung des Puffervolumens, dass der Aktor den Produktstrom aus dem Puffervolumen heraus fördert.

Erfindungsgemäß werden alle Elemente zur Durchführung des Gesamtverfahrens in Units unterteilt. Bevorzugt werden die einzelnen verfahrenstechnischen Schritte des Gesamtprozess als Unit bezeichnet. Durch die Zuordnung der Elemente in Units kann eine Modularität der Produktionsanlage erzeugt werden. Es ist möglich einzelne Prozessschritte auszutauschen, hinzuzufügen oder deren Reihenfolge zu ändern. Die Steuerung / Regelung, also Prozessleitung einer Unit greift hierbei erfindungsgemäß, mit der Ausnahme von Notabschaltungen, nur auf Unitinterne Elemente zu.

Erfindungsgemäß wird als Unit eine Vorrichtung oder Teile einer Vorrichtung zur Durchführung eines verfahrenstechnischen Schrittes bezeichnet. Eine Unit weist im Sinne der Anmeldung eine oder mehrere der folgenden Einheiten auf:
- VTE, die Verfahrens-Technische Einheit, umfasst die Elemente zur Durchführung des Schrittes (auch VT-Elemente), typicherweise Schläuche, Filter, Chromatographie-Säulen, Behälter, usw., die mit dem Leitsystem nicht verbunden sind.
- STE, die Service Technische Einheit, umfasst alle Sensoren und Aktoren der Unit (auch STE-Komponenten genannt). Diese sind über eine RIO mit dem Leitsystem verbunden. Aktoren der STE können bspw. Pumpenmotor, Ventile sein und Sensoren z. B. UV-Messung, Drucksensor oder Waage usw. sein.
- Eine Einheit zur Datenerfassung und -verarbeitung, im einfachsten Fall eine Remote I/O, oder auch eine lokale Intelligenz, z.B. Speicherprogrammierbare Steuerung (SPS) oder PCbasiertes System mit I/O-Ebene. Auf der lokalen Steuerung ist die Basisautomatisierung der Unit implementiert. Beide Systemvarianten werden im nachfolgenden RIO genannt.

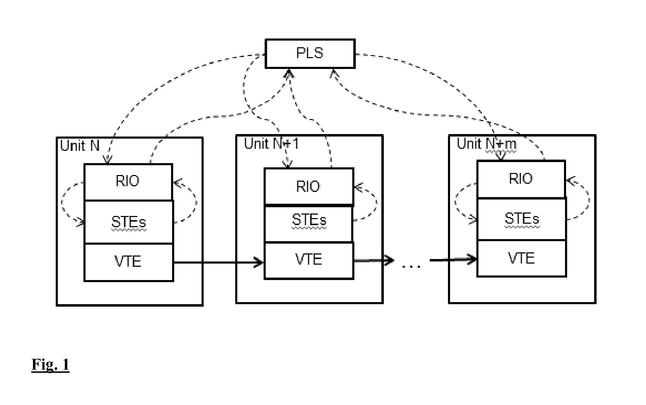

Figur 1 zeigt eine schematische Darstellung einer besonderen Ausführungsform des allgemeinen Aufbaus einer Unit, deren RIO/STE und VTE sowie deren Verbindung mit dem PLS (Regler nicht einzeln dargestellt) ohne sich darauf zu beschränken.

Die Zustandsgröße eines VTE wird von einem oder mehreren Sensoren der zugehörigen STE ermittelt, wie zum Beispiel der Füllstand eines Behälters mit einer Waage bzw. der Druck in einem Filter durch einen Drucksensor. Der STE-Sensor gibt das entsprechende Signal an die RIO, welche dieses an das Leitsystem überträgt. Bevorzugt werden die Signale der STE über die RIO gebündelt und an das Prozessleitsystem übertragen, wo die entsprechenden Stellwerte berechnet werden.

Das Leitsystem verarbeitet die Signale, und berechnet entsprechende Steuersignale, welche über die RIO an die verbundenen STE-Aktoren (z.B. Motor einer Pumpe) weitergegeben werden. Die entsprechenden STE-Aktoren wirken nun auf die VTE-Elemente, welche wiederum auf die STE-Sensoren rückwirken. Zusammenfassend bilden STE-Sensoren, Regler und STE-Aktoren in ihrem Zusammenspiel einen geschlossenen Wirkungsablauf zur Regelung der physikalischen Zustandsgröße. In der bevorzugten Ausführungsform dienen Sensoren einer STE lediglich der Ermittlung aller Zustandsgrößen der VTE derselben Unit und fuhren nur zur Steuerung / Regelung der Aktoren derselben STE.

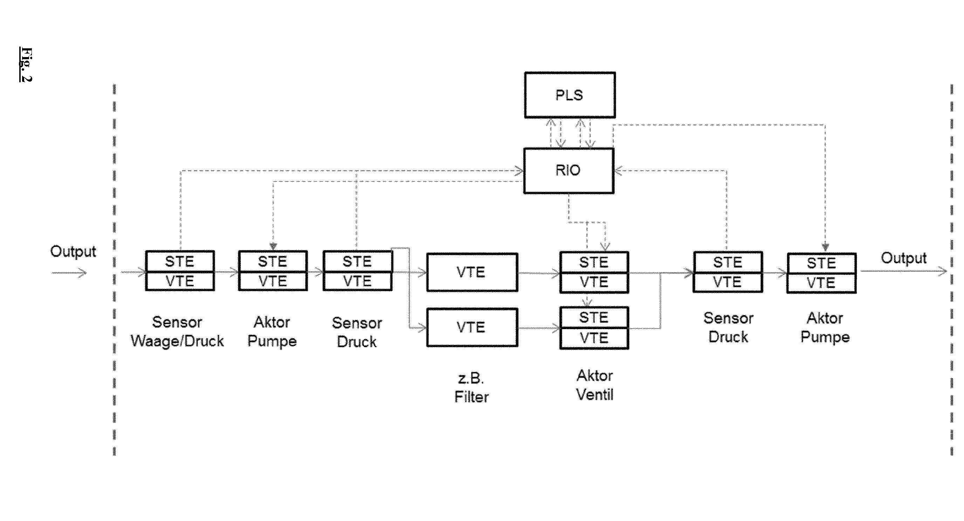

Figur 2 beschreibt exemplarisch den detaillierten Aufbau einer Unit und deren Elemente, sowie deren Verbindungen zum PLS als zentrales Leitsystem (Regler nicht dargestellt), ohne sich darauf zu beschränken. Aus der vorherigen Unit strömt ein Output als Input in das Puffervolumen (VTE-Element) der Unit. Der Zustand des VTE-Elements wird von einem STE-Sensor erfasst, dessen Signale durch die RIO an das PLS weitergeleitet werden. Das PLS sendet ein Signal an die RIO, welche ein Steuersignal an den Motor (STE-Aktor) der Pumpe (VTE-Element) gibt. Der Produktstrom wird weiter über Schläuche (VTE-Elemente) in den Drucksensor (STE-Sensor) geleitet. Das Drucksignal wird in der RIO aufgenommen und an das PLS geleitet.

Ist die VTE beispielsweise ein Filter, wird der Produktstrom über einen ersten Filter geleitet. Stellt die PLS eine Überschreitung eines bestimmten Drucklevels vor dem Filter fest, werden über die RIO Steuersignale an Ventile (STE-Aktor) gesendet, die typischerweise einen automatischen Wechsel des Filters zulassen.
Ist die VTE beispielsweise eine Chromatographiesäule (VTE-Element), würde nach einem bestimmten Aufgabevolumen auf die Säule ein Wechsel der Säulen erfolgen. Als STE kann in diesem Fall ein Durchflusssensor verwendet werden, dessen Daten zeitlich zum Aufgabevolumen integriert werden können. Um die Beladung von Produktmolekülen auf die Säule zu steuern, kann alternativ ein Sensor zur Konzentrationsbestimmung verwendet werden, wie z.B. UV, IR... Die Integration von Flusssignal * Konzentrationssignal ergibt dann die Beladung, die bei Überschreitung analog zum Wechsel der Chromatographiesäulen führen würde.

In dieser bevorzugten Ausführungsform sind die miteinander auf die Regelgröße, insbesondere das Puffervolumen, wirkende Sensoren, Regler und Aktoren in derselben Unit zugeordnet. Zusammenfassend geht der Informationsfluss zur Beförderung des Produktstroms somit üblicherweise entlang der Kette STE_{N} Sensor →RIO_{N}→PLS→RIO→STE_{N} Aktor. Der Produktstrom verläuft entlang der Kette VTE_{N}→VTE_{N+1}→VTE_{N+2} usw.
Alternativ können die Sensoren und / oder Aktoren (STE-Aktoren) zur Regelung des Puffervolumens einer benachbarten (vor- oder nachgeschalteten) Unit zugeordnet sein. In diesem Fall geht der Informationsfluss zur Beförderung des Produktstroms zum Beispiel entlang der Kette STE_{N} Sensor→RIO_{N}→PLS→RIO_{N+1}→STE_{N+1} Aktor; der Produktstrom verläuft ebenfalls entlang der Kette VTE_{N}→VTE_{N+1}.

Erfindungsgemäß umfasst die Produktionsanlage mehrere Units, die in Master Units und Slave Units unterteilt sind.

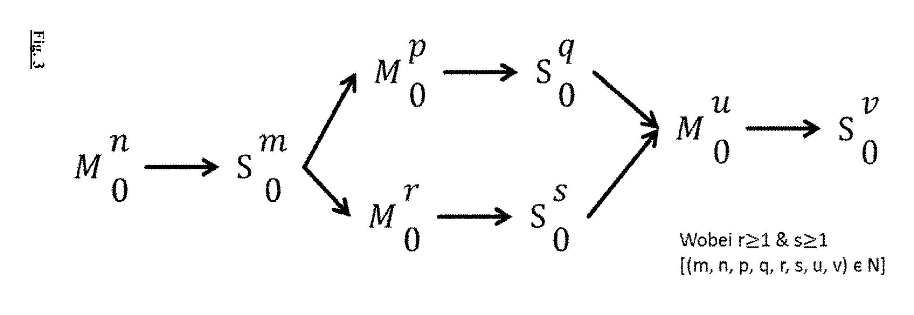

Figur 3 zeigt allgemein die möglichen Anordnungen von Master und / oder Slave Units in der erfindungsgemäßen Produktionsanlage.

Die Figuren 4A, 4B und 4C veranschaulichen schematisch den Aufbau von Slave Units (4A, 4B) sowie einer Slave Unit, die temporär als Master Unit betrieben werden kann (4C).

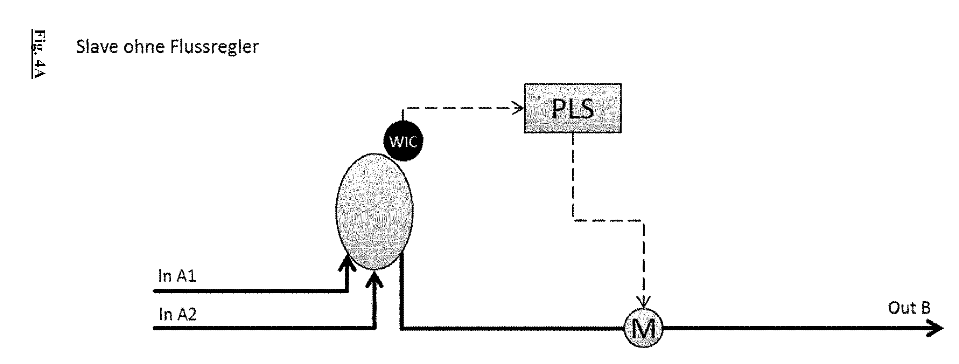

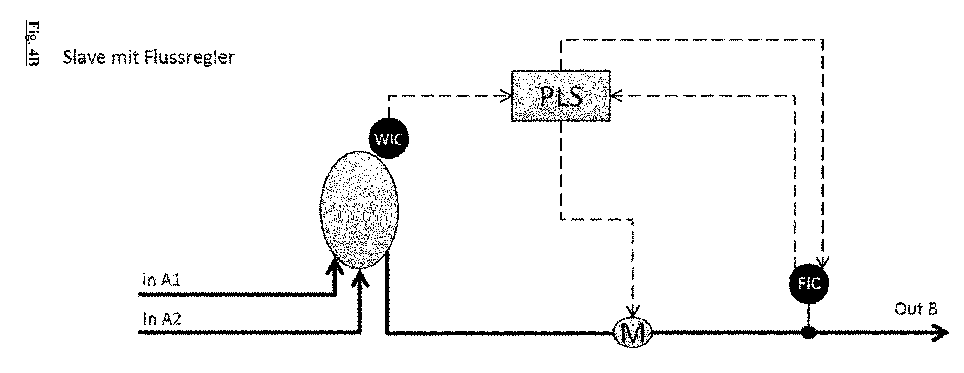

Erfindungsgemäß sind Master Unit und Slave Unit folgendermaßen abhängig von ihren Regelungs- bzw. Steuerverhalten definiert:
- Der Soll-Wert der Flussrate einer Master Unit ergibt sich nicht über die Regelung der Zustandsgröße eines Puffervolumens. Üblicherweise wird er durch das Leitsystem vorgegeben. Eine Master Unit muss sich nicht einer anderen Unit hinsichtlich ihrer Flussrate anpassen. Erfindungsgemäß umfasst eine Master Unit einen oder mehrere Aktoren und eine Leitung zur Beförderung des Produktstroms sowie eine RIO. Sensoren z. B. zur Messung und Regelung der Flussrate sind optional aber bevorzugt. Wenn Sensoren z. B. zur Messung und Regelung der Flussrate der Master Unit verwendet werden, ist die Master Unit üblicherweise mit mindestens einem Regler verbunden. Dieser Regler kann bevorzugt Teil des Leitsystems, d. h. in einer zentralen Regelung, oder alternativ Teil einer lokalen speicherprogrammierbaren Steuerung (SPS) in einer dezentralen Regelung sein. Typischerweise ist eine Master Unit eine Chromatographie-Unit, eine Virusinaktivierungsunit und / oder eine Filtrationsunit.
- Der Soll-Wert der Flussrate einer Slave Unit ergibt sich über die Regelung der Zustandsgröße eines Puffervolumens in derselben oder in einer entlang des Produktstroms benachbarten Unit. Das heißt, eine Slave Unit muss sich einer anderen Unit hinsichtlich ihrer Flussrate anpassen. Eine Slave Unit weist für die Beeinflussung ihres Puffervolumens einen geschlossenen Wirkungsablauf auf, der mittels eines STE-Sensors zur Überwachung des Puffervolumens (als WIC dargestellt), eines Reglers und eines STE-Aktors zur Beeinflussung des Puffervolumens (M) - alle zusammen als Elemente zur Beeinflussung der Puffervolumens genannt - erreicht wird (Fig. 4A). Zur Regelung der Zustandsgröße des Puffervolumens kann der STE-Sensor zur Überwachung des Puffervolumens (WIC) mit einem Sensor zur Flussregelung kombiniert werden (FIC) wie in Fig 4B dargestellt.

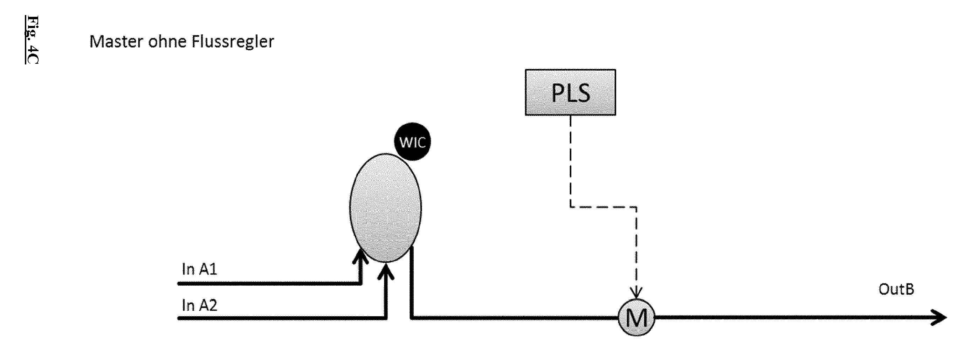

Der Soll-Wert der Flussrate einer Slave Unit kann unter Umständen, üblicherweise temporär (z. B. beim Ausfall/Pausieren der vorgelagerten Master Unit), wie bei einer Master Unit gesteuert werden (Fig. 4C).

Mit Überwachung bzw. Beeinflussung des Puffervolumens ist im Sinne der Anmeldung die Überwachung bzw. Beeinflussung der Zustandsgröße des Puffervolumens gemeint.

Im Sinne der Erfindung wird typischerweise der Produktstrom, der aus dem Puffervolumen jeder Slave Unit herausgeht (Outputstrom B), derart kontrolliert, dass trotz Schwankungen einer oder mehreren Inputströme (Inputstrom A1, A2) die Zustandsgröße des Puffervolumens im zeitlichen Mittel konstant bleibt. Der Outputstrom B muss nicht immer exakt die Summe der Inputströme A1 und A2 sein.

Typischerweise sind alle STE-Elemente zur Beeinflussung des Puffervolumens derselben Unit zugeordnet. Mit anderen Worten umfasst in der bevorzugten Ausführungsform eine Slave Unit mindestens ein Puffervolumen, mindestens einen Sensor (STE-Sensor) zur Überwachung des Puffervolumens und einen oder mehreren Aktoren (STE-Aktoren) zur Beeinflussung des Puffervolumens. Die Sensoren zur Überwachung und Aktoren zur Beeinflussung des Puffervolumens sind mit mindestens einem Regler verbunden. Mindestens einer dieser Regler regelt die Zustandsgröße des Puffervolumens. Dieser Regler kann Teil des Leitsystems (zentrale Regelung) oder Teil einer lokalen SPS (dezentrale Regelung) sein.

Alternativ können aber die Puffervolumen, Sensoren zur Überwachung und / oder Aktoren zur Beeinflussung des Puffervolumens einer benachbarten (vor- oder nachgeschalteten) Unit zugeordnet sein. Z. B. kann eine Master Unit mindestens ein Puffervolumen zur Regelung der nachgeschalteten Unit und mindestens einen Sensor (STE-Sensor) zur Überwachung des Puffervolumens umfassen; der entsprechende Aktor zur Beeinflussung des Puffervolumens ist dann der nachgeschalteten Slave Unit zugeordnet. Eine solche Zuordnung erfolgt typischerweise, wenn eine Chromatographieanlage als Slave Unit betrieben werden soll oder wenn aus platztechnischen Gründen das Puffervolumen auf dem entsprechenden Skid nicht untergebracht werden kann.

Zusammenfassend umfasst die erfindungsgemäße Produktionsanlage für jede Slave Unit mindestens ein Puffervolumen zur Aufnahme des Produktstroms sowie ein oder mehrere Sensoren, Regler und Aktoren (STE-Aktoren) zur Regelung des Puffervolumens entweder in der gleichen Unit oder in einer benachbarten (d. h. entlang des Produktstroms vor- oder nachgeschalteten) Unit.

Bevorzugt wird eine Quellregelung innerhalb der Slave Units eingesetzt, d. h. dass das Puffervolumen die Quelle ist, aus der der Aktor den Produktstrom herausfördert. Daher wird in diesem Fall eine Master Unit am Anfang der Anlage verwendet.

Alternativ kann eine Zielregelung innerhalb der Slave Unit eingesetzt werden, bei der das Puffervolumen das Ziel ist, in das der Aktor den Produktstrom hineinfördert.

Für den sicheren Betrieb, d. h. um die Abschaltung einer Unit während des Betriebs der Anlage zu ermöglichen, ermöglicht das Leitsystem typischerweise eine zentrale Überwachung der Puffervolumen und ermöglicht die Abschaltung einer Unit bei Bedarf (Puffervolumen zu voll oder zu leer); jede Master und jede Slave Unit ist mit dem Leitsystem verbunden.

Das gesamte Leitsystem kann eine Kombination von zentralen und dezentralen Regelungen sein. Typische Units mit lokaler Regelung sind Chromatographieanlagen.

Erfindungsgemäß kann das Puffervolumen in einer Unit durch die Anwendung eines dehnbaren Schlauchs oder eines Behälters generiert werden. Die Größe des Puffervolumens kann dann über den Druck oder z.B. über das Gewicht ermittelt werden. Der STE-Sensor zur Überwachung des Puffervolumens ist typischerweise ein Füllstandsensor wie z. B. ein Drucksensor, eine Waage, ein optischer Sensor, usw.

Bevorzugt weist jeder Behälter eine Belüftung - ein Ventil oder einen Belüftungsfilter - auf.

Bevorzugt wird ein dehnbarer Schlauch verwendet. Als dehnbarer Schlauch wurde beispielsweise ein Silikonschlauch des Typs SaniPure® in einer Testanlage verwendet. Als dehnbarer Schlauch werden Pharmed®-BPT (Silikonschlauch), C-Flex-374® (thermoplastischer Schlauch), oder SaniPure® der Firma Saint-Gobain Performance Plastics genannt ohne sich darauf zu begrenzen. Typischerweise wird zur Überwachung der Dehnung des Schlauches, und damit des Puffervolumens, ein Drucksensor verwendet. Das Überlaufen oder Leerlaufen des Puffervolumens wird dadurch verhindert, dass im Leitsystem ein erlaubten Druckbereich für das Puffervolumens definiert werden, so dass beim Überschreiten der Druckobergrenze der Aktor zur Beförderung des Produktstroms in das Puffervolumen abgeschaltet wird. Beim Unterschreiten der Untergrenze wird der Aktor zur Beförderung des Produktstroms aus dem Puffervolumen abgeschaltet. Ein dehnbarer Schlauch wird beispielsweise als Puffervolumen in einer Deadend-Filtration, die einer anderen Deadend-Filtration nachgeschaltet ist, bevorzugt verwendet. Hiermit können Totvolumen in der Anlage reduziert werden.

In einer alternativen Ausführungsform wird zur Regelung des Puffervolumens eine Behälter-Füllstandssensor Kombination, insbesondere eine Behälter-Waage Kombination verwendet.

Beide Ausführungsformen ermöglichen die Flussratenkompensierung zwischen zwei Units, auch bei einem Halt oder einem kurzzeitigen Stopp einer der beiden Units.

Verschiedene Kombinationen von Puffervolumen und Füllstandsensoren können in der gleichen Produktionsanlage verwendet werden.

Über das Leitsystem wird der Füllstand in dem Puffervolumen auf einen jeweiligen Sollwert geregelt. In der Testanlage wurden die Sollfüllstände der Behälter typischerweise derart eingestellt, dass die mittlere Verweilzeit zwischen 2 min und 4 h liegt, bevorzugt bei 20 min. Der

Sollwert bei der Druckregelung lag zwischen -0,5 bar und 2 bar, bevorzugt -100 bis 200 mbar, besonders bevorzugt 10 bis 50 mbar gegenüber Umgebungsdruck.

Im Leitsystem wird die Richtung des Informationsflusses zwischen den Elementen, STE-Sensoren, Regler und STE-Aktoren, die zur Regelung eines Puffervolumens beitragen, nach den oben genannten Definitionen festgelegt und dadurch die Units in Master oder Slave Units unterteilt. Dies kann durch den Nutzer über einen User-Interface oder in der Konfiguration des Leitsystems vorgenommen werden.

Bevorzugt ist das Leitsystem für eine automatische Kompatibilitätsprüfung der manuellen Unterteilung der Units nach den oben genannten Definitionen programmiert.

Es wird vermerkt, dass für die Zuordnung der Elemente zur Regelung des Puffervolumens in einer oder benachbarten Unit und / oder für die Festlegung der Richtung des Informationsflusses zwischen den Elementen - STE-Sensoren, Regler und STE-Aktoren - zur Regelung eines Puffervolumens jeweils nur die Elemente jedes geschlossenen Wirkungsablaufs in Betracht bezogen werden. Die Zuordnung von STE-Elementen entlang des Produktstroms zu einer Unit sind Teil des modularen Aufbaus der Produktionsanlage. Die Einzelbetrachtung von geschlossenen Wirkungsabläufen zur Regelung der Puffervolumen im Zusammenhang mit dem kontinuierlichen Produktstrom und dessen Flussraten ermöglicht den erfindungsgemäßen modularen Aufbau der Steuerung/Regelung der Produktionsanlage in Units.

Erster Gegenstand der Anmeldung ist daher eine Produktionsanlage zur kontinuierlichen Herstellung und / oder Aufbereitung von biopharmazeutischen Produkten mit mindestens zwei in Serie miteinander verbundenen Units zur Durchführung von mindestens zwei Downstream- und / oder Upstream-Schritten, wobei die Produktionsanlage umfasst:
- Mindestens eine Slave Unit und mindestens eine Master Unit,
- wobei jede Slave Unit mit mindestens einem Puffervolumen entweder in der gleichen Unit oder in einer entlang des Produktstroms benachbarten Unit verbunden ist und ein oder mehrere Sensoren zur Überwachung des Puffervolumens und ein oder mehrere Aktoren zur Beeinflussung des Puffervolumens aufweist und, wobei die Zustandsgröße jedes Puffervolumens mit Hilfe des Sensors und des Aktors verbunden mit mindestens einem Regler in einem geschlossenen Wirkungsablauf geregelt ist,
- wobei eine Master Unit mindestens eine Vorrichtung zur Beförderung des Produktstroms umfasst und dadurch gekennzeichnet ist, dass ihre Flussrate nicht über die Regelung der Zustandsgröße eines Puffervolumens kontrolliert wird,
- und wobei, wenn die Master Unit mit einer oder mehrerer Slave Units benachbart ist, sie mit dem Puffervolumen jeder Slave Unit verbunden ist, und
wobei im Fall mehrerer Master Units mindestens ein Auxiliarystrom zwischen zwei Flussraten-bestimmenden Aktoren der Master Units vorhanden ist.

Bevorzugt sind ein oder mehrere der Regler Komponenten eines Leitsystems, vornehmlich eines Prozessleitsystems.

Um die Abschaltung einer Master Unit während des Betriebs zu ermöglichen, ist jede Master Unit bevorzugt mit dem Leitsystem verbunden.

Weiterer Gegenstand der Anmeldung ist ein computer-implementiertes Verfahren zur Prozesssteuerung der erfindungsgemäßen Produktionsanlage, wobei:
- die Werte der Zustandsgröße der Puffervolumen und der Flussrate in der Produktionsanlage, durch folgende Angaben festgelegt werden:
   ∘ Reihenfolge der Units entlang des Produktstroms wird angegeben,
   ∘ ein Soll-Wert für die Flussrate jeder Master Unit wird festgelegt,
   ∘ ein Soll-Wert für die Zustandsgröße jedes Puffervolumens wird festgelegt,
   ∘ für jeden geschlossenen Wirkungsablaufs werden die Verbindung der Regler mit den Sensoren zur Überwachung des Puffervolumens und mit den Aktoren zur Beeinflussung des Puffervolumens sowie ggf. deren Verbindung untereinander festgelegt,
   ∘ eine Parametrisierung der Regler wird vorgenommen,

Das erfindungsgemäße Verfahren umfasst für den Betrieb der Produktionsanlage folgende Schritte:
a) Der Soll-Wert für die Flussrate der Master Units wird von dem Leitsystem an einen Aktor zur Steuerung der Flussrate in der Master Unit übertragen, mit der Maßnahme, dass im Fall mehrerer Master Units ein Auxiliarystrom geöffnet wird, und
b) Der Ist-Wert der Zustandsgröße jedes Puffervolumens wird durch den entsprechenden Sensor zur Überwachung des jeweiligen Puffervolumens ermittelt, an den im jeweils geschlossenen Wirkungsablauf verbundenen Regler weitergeleitet und dort mit dem jeweils entsprechenden Soll-Wert verglichen,
c) Die jeweiligen Steuersignale werden berechnet und jeweils an die im geschlossenen Wirkungsablauf verbundenen Aktoren zur Beeinflussung der Puffervolumen übertragen,
d) Die Aktoren zur Beeinflussung des Puffervolumens rückwirken auf die Sensoren zur Überwachung des Puffervolumens und
e) Schritte b) bis d) werden wiederholt, bis die Produktionsanlage abgeschaltet oder abgefahren ist.

Bevorzugt werden zusätzlich Abschaltbedingungen durch folgende Angabe definiert:
∘ ein Maximal- und / oder Minimal-Wert für die Zustandsgröße jedes Puffervolumens wird festgelegt, bevorzugt beides,
∘ ein Maximal- und / oder Minimalwert für die Flussrate jeder Master Unit wird festgelegt, bevorzugt beides.

Weiterer Gegenstand ist ein Computer Programm zur Durchführung des oben genannten Verfahrens.

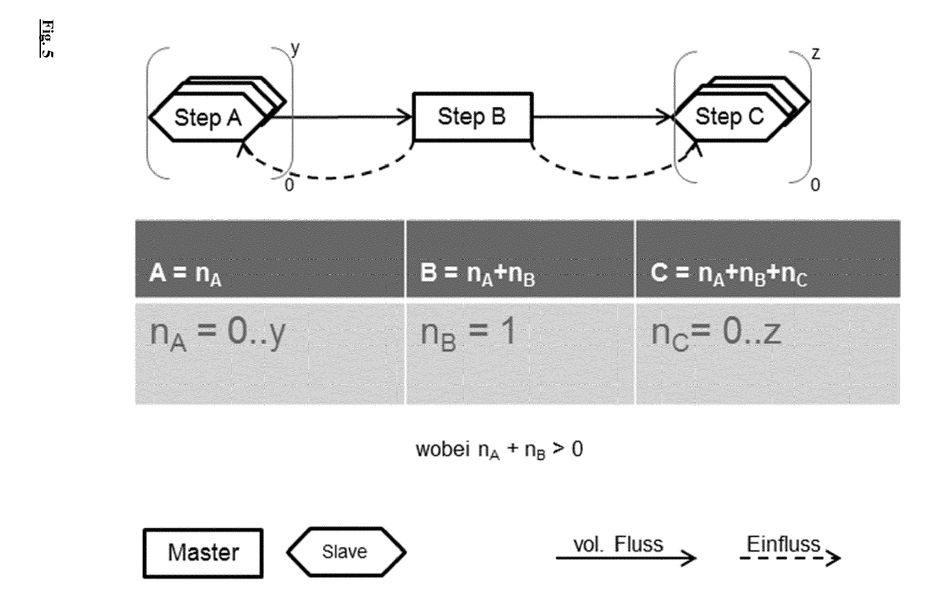

Figur 5 zeigt eine schematische Darstellung einer Produktionsanlage mit nur einer Master Unit (Step C, n_{C}=1). Richtung des Produktstroms und des Informationsflusses in der Anlage sind entsprechend ebenfalls definiert worden.

Die Anlage kann n_{A} =0 bis y Slave Units - hier als (Step A)_{0..y} zusammengefasst - umfassen.

Ebenso kann die Anlage nc =0 bis z Slave Units, hier als (Step C)_{0...z} zusammengefasst, umfassen.

Die Prozessschrittnummer (y bzw. z) stellt die letzte Prozessschrittnummer in der Reihe dar.

In dieser Konfiguration kann eine Slave Unit (Step A bzw. Step C) jeweils als einzelne Unit am Anfang und / oder Ende der Anlage stehen.

Typischerweise ist ein Chromatographie-Schritt eine Master Unit. Mehrere ChromatographieSchritte können alle als Master Units agieren vorausgesetzt, ein Auxilarystrom ist zwischen jeweils zwei Master Units vorhanden. "Zwischen zwei Master Units" bedeutet hier hinter der Pumpe zur Förderung des Produktstroms aus der ersten Master Unit und der ersten Pumpe zu Förderung des Produktstroms in der Master Unit 2.

Alternativ wird nur eine Chromatographie-Unit als Master Unit betrieben, die anderen Chromatographie-Units werden jeweils mit Hilfe eines Puffervolumens als Slave Units und bevorzugt mit einer Hystereseregelung (zentral oder lokal) geregelt.

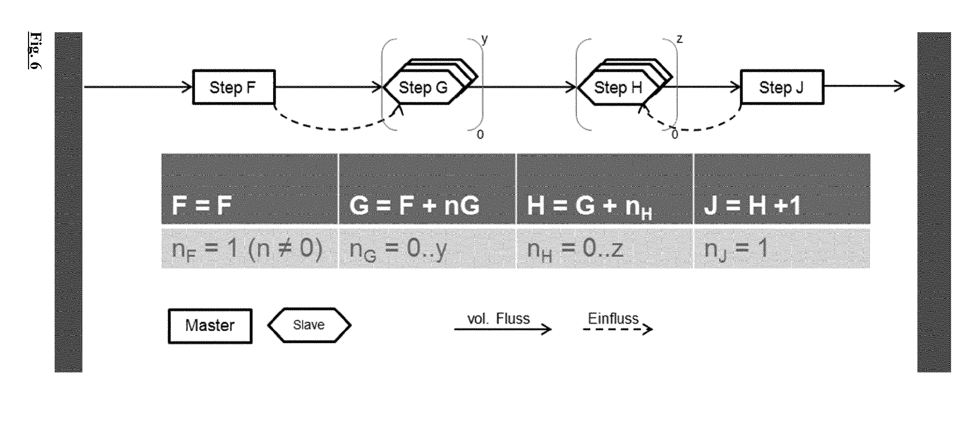

Fig. 6 zeigt eine schematische Darstellung eines Teils einer weiteren Produktionsanlage umfassend zwei Master Units (Step F, nF=1 und Step J, nJ=1). Fig. 6 bildet nur den Teil zwischen den Master Units ab. Das Gesamtbild des Prozesses ergibt sich aus der Kombination mit Fig. 5 zur Regelung des Anfangs und Ende der Prozessanlage.

Es gibt für den Gesamtprozess immer eine Master Flussrate (PF), die extern oder durch eine Master Unit vorgeben wird, bzw. von der ersten Master Unit in Produktstromrichtung, wenn mehrere vorkommen.

Zwischen zwei Master Units muss mindestens ein Auxiliarystrom (in Fig nicht dargestellt) vorhanden sein, da es nicht möglich ist, zwei Master Units mit genau gleicher Flussrate zu steuern. Der Auxilarystrom führt Flüssigkeit in den Produktstrom herein oder aus dem Produktstrom heraus (Konzentrierung). Der Auxilarystrom kompensiert die Differenz zwischen der Master Flussrate, in Figur 6 vorgegeben durch Master Unit F, und der Flussrate der nachgeschalteten Master Unit J.

Auxilarystrom im Sinne der Anmeldung bezeichnet ein nicht produktbehafteter (oder abfallproduktbehafteter) Strom, welcher in den Produktstrom hinein oder herausgeführt wird. Auxilaryströme, die dem Produktstrom hineingeführt werden, können geregelt sein. Typischerweise umfasst eine der Master Unit in dieser Ausführungsform der Produktionsanlage ein STE-Sensor zur Messung des Auxilarystroms und ein STE-Aktor zur Regelung und Steuerung des Auxiliarystroms, sowie VTE-Elemente zur Zufuhr bzw. Abfuhr eines Auxiliarystroms (die als AUX-VTE-Elemente zusammengefasst werden). Auxilaryströme, die aus dem Produktstrom hinausgeführt werden, werden üblicherweise nicht geregelt.

Wird bspw. einer kontinuierlichen Elution von einer Protein A-Chromatographie (Master 1 mit Fluss F1) eine kontinuierliche Virusinaktivierung mit konstantem Eingangsstrom (Master 2 mit Fluss F2) nachgeschaltet, so wird ein Auxillary (F3) benötigt um die Flussratendifferenz zu kompensieren, da F2>F ist. F2<F ist nicht sinnvoll da es zu Produktverlust führt, und F1=F2 ist technisch ohne Regelung nicht möglich. Flussraten F1 und F2 werden nicht geregelt, sondern nur gesteuert. Fluss F3 ergibt sich entweder von allein (F3=F2-F1), oder kann durch eine Regelung des Füllstandes oder Drucks gesteuert werden. Bevorzugt ergibt sich der Fluss F3 von allein. Obwohl die erfindungsgemäße Anlage mindestens eine Master und mindestens eine Slave Unit aufweist, ist die Nutzung eines Auxilarystroms auf eine Anlage, die nur Master Units umfasst, übertragbar.

Eine weitere typische Master Unit ist die kontinuierliche Virus Inaktivierung nach PCT/EP2015/054698. Umfasst die Anlage eine Chromatographie Unit und eine kontinuierliche Virus Inaktivierung kann ein Auxiliarystrom zwischen den Master Units verwendet werden. In dieser Ausführungsform wird der Chromagraphie Unit ein Auxiliary Strom dem Produktstrom vor der kontinuierliche Virus Inaktivierung immer (während des Betriebs und Anhaltens) hinzugefügt. Um dies zu vermeiden wird bevorzugt die Chromatographie-Unit als Master Unit und die kontinuierliche Virus Inaktivierung als Slave Unit betrieben. Dabei ist zu beachten, dass wenn die Chromatographie-Unit (Master Unit) angehalten ist, die kontinuierliche Virus Inaktivierung, als zeitkritischer Schritt, als Master Unit betrieben werden muss. Dies wird dadurch erreicht, dass sowohl ein Auxiliary Strom für den Betrieb der Unit zur kontinuierlichen Virus Inaktivierung als Master Unit, als auch ein Puffervolumen für den Betrieb der Unit zur kontinuierlichen Virus Inaktivierung als Slave Unit, zwischen der Chromatographie Unit und die Unit zur kontinuierlichen Virus Inaktivierung vorhanden sind.

In einer bevorzugten Ausführungsform der Produktionsanlage werden die Units zur Durchführung der Schritte in Units folgendermaßen betrieben:
- Perfusionskultur und Zellrückhaltesystem bilden typischerweise eine Unit, die typischerweise als Master Unit betrieben wird,
- Konzentrierung und eine direkt nachgelagerte Dialyse können ebenfalls zusammen eine Unit bilden, die als Slave Unit betrieben wird. Vorzugsweise wird aber zwischen Konzentrierung und Dialyse eine Filtration durchgeführt. In diesem Fall bilden sie getrennte Slave Units.
- Chromatographie Units werden typischerweise als Master Units betrieben. Eine Chromatographie Unit kann aber auch als Slave Unit betrieben werden, wenn die Software zur Steuerung der Chromatographie dies ermöglicht, d. h. die Chromatographie automatisch mit mindestens zwei unterschiedlichen Geschwindigkeiten gefahren werden kann.
- Homogenisierung, Virus Inaktivierung und Neutralisation bilden vorzugsweise zusammen eine Unit, die typischerweise als Slave Unit, aber bevorzugt bei Bedarf temporär als Master Unit, betrieben wird.
- Filtrationen - zur Zellabtrennung, Filtration zur Bioburden Reduktion oder Partikelabtrennung bzw. Virenfiltration - sind typischerweise Slave Units.
- Verweilzeitglieder für Reaktion wie z.B. Fällungen oder auch Kristallisationen sind typischerweise Slave Units, werden bevorzugt aber in andere Units integriert. Für die kontinuierliche Fahrweise wird ein Verweilzeitelement, z.B. Schlauch, bevorzugt gewickelter Schlauch, besonders bevorzugt ein Coiled Flow Inverter (CFI) verwendet.
- Konditionierungsglieder für die Parametereinstellung des Produktstroms wie beispielsweise pH-, Leitfähigkeitswerte sind typischerweise Slave Units, werden bevorzugt aber in andere Units integriert. Bevorzugt erfolgt das Konditionieren in einer Konditionierungsschleife, welche an das Puffervolumen angeschlossen ist.

Die Units der Produktionsanlage können alle kontinuierlich betrieben werden. In dieser Ausführungsform wird die Virus Filtration bevorzugt als letzter Schritt vor einer Bioburden Reduzierung oder als letzter Prozessschritt durchgeführt. Dies ermöglicht bei Bedarf eine erneute Virus Filtration des Produktstroms. Dies hat den weiteren Vorteil, dass bei Bedarf die Betriebsweise der Units - Viren Filtration mit / ohne Bioburden Reduzierung - von kontinuierlich auf Batch geändert werden kann.

Alternativ können einzelne Units absatzweise betrieben werden. Zum Beispiel können alle Schritte bis zur Viren Inaktivieren kontinuierlich betrieben werden, die Viren Inaktivierung im Batch gefahren werden und die weiteren Schritte wieder kontinuierlich gefahren werden, wobei der Puffervolumen so konfiguriert werden muss, dass der kontinuierliche Betrieb der vor-/nachgelagerten Units gewährleistet ist.

In der erfindungsgemäßen Anlage beträgt der Soll-Wert der Flussrate von produktbehaftete Volumenströme üblicherweise 0,001 bis 10 L/Minute, bevorzugt 0,01 bis 5 L/min, besonders bevorzugt 0,05 bis 1 L/min.

Die Messung von Flussraten insbesondere von ≤50 ml/min ist in einer kontinuierlich betriebenen Anlage eine Herausforderung. Es wurde festgestellt, dass diese Messung mit Hilfe von kommerziell erhältlichen, autoklavierbaren oder gamma-sterilisierbar Einweg-Durchflussmessern nicht möglich ist. Diese Messung kann in einer Anlage mit flexiblen Leitungen, in denen einen Flüssigkeitsstrom befördert wird, durch die Anwendung einer kompensierenden Flussratenmessung gelöst werden. Diese wird durch eine Kombination einer kompensierenden Pumpe, eines Drucksensors und eines Reglers mit einem gewünschten Soll-Druck gelöst. Die Druckdifferenz zwischen Ein- und Ausgang der kompensierenden Pumpe wird nahezu konstant gehalten. Bevorzugt ist diese Differenz null, besonders bevorzugt entspricht der Druck vor und hinter der kompensierenden Pumpe jeweils dem Umgebungsdruck. Bei Abweichungen des Ist-Drucks vom Solldruck werden die Drehzahl und damit die Förderleistung der kompensierenden Pumpe entsprechend angepasst. Über die Messung der Drehzahl der kompensierenden Pumpe und den Fördervolumen pro Umdrehung kann schließlich die Flussrate errechnet werden (=kompensierende Flussratenmessung).

Die Größe des Puffervolumens hängt von den Flussraten und der Trägheit der Regelung ab. Erfordert eine Unit ein regelmäßiges Abschalten für die Wartung eines VTE-Elements wird vorzugsweise ein größeres Puffervolumen in Form eines Behälters verwendet. Typische solche Units sind Chromatographie.

Typischerweise weist ein Behälter keinen Rührer aus. Ist eine Mischung des Inhalts des Behälters erforderlich, kann ein Rührer verwendet werden, bevorzugt aber erfolgt die Mischung mit Hilfe einer Zirkulation (Leitung und Pumpe).

Zur Veranschaulichung des erfindungsgemäßen Verfahrens wird die Konfiguration verschiedenen PLS für Anlagen zum Upstream und Downstream Processing oder nur Downstream Processing eines Produktstroms aus einem Fermenter schematisch dargestellt. Diese Konfigurationen sind exemplarisch und stellen keine Begrenzung des erfindungsgemäßen Verfahrens dar.

In den Figuren wird die Produktionsanlage in Skids unterteilt. Ein Skid ist gemäß dem Stand der Technik eine drei-dimensionale feste Struktur, die als Plattform oder Träger einer Unit dienen kann. Beispiele von Skids sind in den Figuren aufgeführt.

### Beispiele

### 1) Fermentation -> DSP I und DSP II

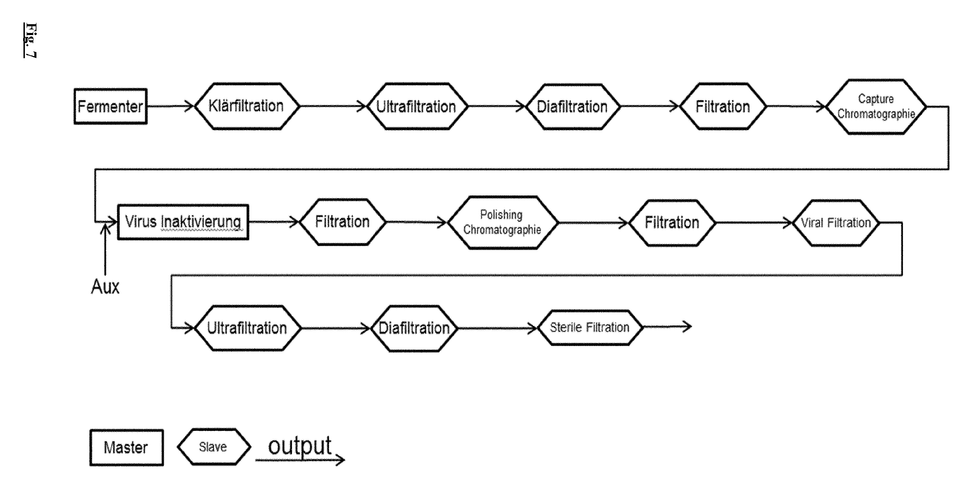

Figur 7 zeigt beispielhaft einen möglichen kontinuierlichen Prozess von der Fermentation bis zur finalen Filtration. Diese Produktionsanlage umfasst zwei Master Units - die Fermentation und die verweilzeitkritische Vireninaktivierung (VI). Um einen im zeitlichen Mittel konstanten Volumenstrom aus der Vireninaktivierung (VI) realisieren zu können, wird nach der Capture Chromatographie, die in diesem Beispiel als Slave betrieben wird, ein Auxiliarystrom (Aux) zugeführt. Die anderen Units sind Slave Units.

### 2) Nur DSP II bei dem gemäß Fig 6, nG=nH=0

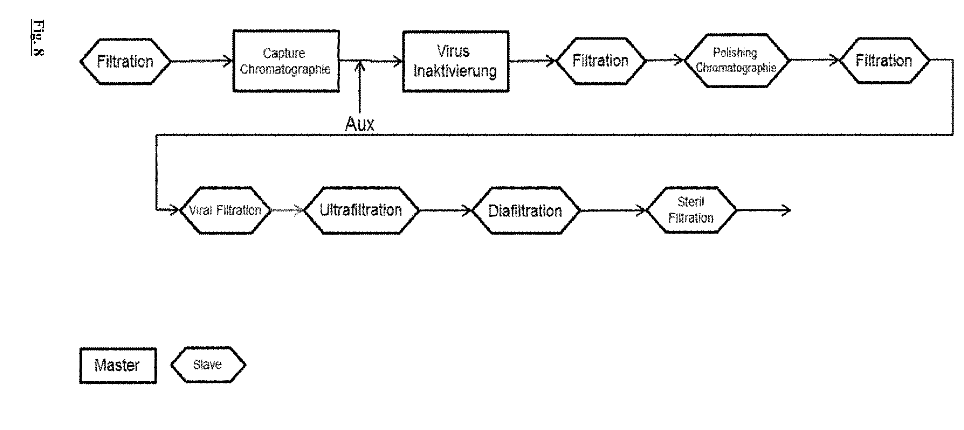

Figur 8 zeigt ein Beispiel, in dem der Downstream Prozess nicht direkt mit der Fermentation gekoppelt ist, wobei die Capture Chromatographie und die Vireninaktivierung (VI) zwei Master Units sind. Um einen konstanten Volumenstrom aus der Capture Chromatographie realisieren zu können, wird nach dieser ein Auxiliarystrom (Aux) zugeführt. Die der Capture Chromatographie vorgelagerte Filtration ist dann eine Slave Unit. Die nachgelagerten Units sind ebenfalls Slave Units.

**Die Arbeiten, die zu dieser Anmeldung geführt haben, wurden gemäß der Finanzhilfevereinbarung "Bio.NRW: MoBiDiK - Modulare Bioproduktion - Disposable und Kontinuierlich" im Rahmen des Europäischen Fonds für regionale Entwicklung (EFRE) gefördert.**

## Patentansprüche

1. Produktionsanlage zur kontinuierlichen Herstellung und / oder Aufbereitung von biopharmazeutischen Produkten mit mindestens zwei in Serie miteinander verbundenen Units zur Durchführung von mindestens zwei Downstream- und / oder Upstream-Schritten, wobei die Produktionsanlage umfasst:
- Mindestens eine Slave Unit und mindestens eine Master Unit,
- wobei jede Slave Unit mit mindestens einem Puffervolumen entweder in der gleichen Unit oder in einer entlang des Produktstroms benachbarten Unit verbunden ist und ein oder mehrere Sensoren zur Überwachung des Puffervolumens und ein oder mehrere Aktoren zur Beeinflussung des Puffervolumens aufweist und, wobei die Zustandsgröße jedes Puffervolumens mit Hilfe des Sensors und des Aktors verbunden mit mindestens einem Regler in einem geschlossenen Wirkungsablauf geregelt ist,
- wobei eine Master Unit mindestens eine Vorrichtung zur Beförderung des Produktstroms umfasst und **dadurch gekennzeichnet ist, dass** ihre Flussrate nicht über die Regelung der Zustandsgröße eines Puffervolumens kontrolliert wird,
- und wobei, wenn die Master Unit mit einer oder mehrerer Slave Units benachbart ist, sie mit dem Puffervolumen jeder Slave Unit verbunden ist, und
wobei im Fall mehrerer Master Units mindestens ein Auxiliarystrom zwischen zwei Flussraten-bestimmenden Aktoren der Master Units vorhanden ist.

2. Produktionsanlage nach Anspruch 1, wobei ein oder mehrere der Regler Komponenten eines Leitsystems, vornehmlich eines Prozessleitsystems sind.

3. Produktionsanlage nach Anspruch 1 oder 2, Master Unit mit dem Leitsystem verbunden ist.

4. Produktionsanlage nach einem der Ansprüche 1 bis 3, wobei die letzte oder die vorletzte Unit entlang des Produktstroms eine Virus Filtration durchführt.

5. Produktionsanlage nach einem der Ansprüche 1 bis 4, umfassend ein oder mehrere Verweilzeitglieder zur Gewährleistung bestimmter Verweilzeiten.

6. Produktionsanlage nach einem der Ansprüche 1 bis 5, umfassend ein oder mehrere Konditionierungsglieder zur Einstellung bestimmter Parameter des Produkstroms.

7. Produktionsanlage nach Anspruch 6, wobei das Konditionierungsglied eine Konditionierungsschleife ist.

8. Produktionsanlage nach Anspruch 7, wobei die Konditionierungsschleife an ein Puffervolumen angeschlossen ist.

9. Produktionsanlage nach einem der Ansprüche 1 bis 8, wobei das Puffervolumen durch einen dehnbaren Schlauch zur Verfügung gestellt wird.

10. Produktionsanlage nach einem der Ansprüche 1 bis 9, wobei das Puffervolumen, die miteinander auf die Zustandsgröße des Puffervolumens wirkenden Sensoren, Regler und Aktoren in derselben Unit zugeordnet sind.

11. Produktionsanlage nach einem der Ansprüche 1 bis 10, wobei die Produktionsanlage flexible Leitungen aufweist, in denen einen Flüssigkeitsstrom befördert wird, der durch die Anwendung einer kompensierenden Flussratenmessung gemessen wird.

12. Computer-implementiertes Verfahren zur Prozesssteuerung der erfindungsgemäßen Produktionsanlage, wobei:
- im Schritt a) die Werte der Zustandsgröße der Puffervolumen und der Flussrate in der Produktionsanlage, durch folgende Angaben festgelegt werden:
∘ Reihenfolge der Units entlang des Produktstroms wird angegeben,
∘ ein Soll-Wert für die Flussrate jeder Master Unit wird festgelegt,
∘ ein Soll-Wert für die Zustandsgröße jedes Puffervolumens wird festgelegt,
∘ für jeden geschlossenen Wirkungsablaufs werden die Verbindung der Regler mit den Sensoren zur Überwachung des Puffervolumens und mit den Aktoren zur Beeinflussung des Puffervolumens sowie ggf. deren Verbindung untereinander festgelegt,
∘ eine Parametrisierung der Regler wird vorgenommen,
und wobei das Verfahren folgende weitere Schritte für den Betrieb der Produktionsanlage umfasst:
b) Der Soll-Wert für die Flussrate der Master Units wird von dem Leitsystem an einen Aktor zur Steuerung der Flussrate in der Master Unit übertragen, mit der Maßnahme, dass im Fall mehrerer Master Units ein Auxiliarystrom geöffnet wird, und
c) Der Ist-Wert der Zustandsgröße jedes Puffervolumens wird durch den entsprechenden Sensor zur Überwachung des jeweiligen Puffervolumens ermittelt, an den im jeweils geschlossenen Wirkungsablauf verbundenen Regler weitergeleitet und dort mit dem jeweils entsprechenden Soll-Wert verglichen,
d) Die jeweiligen Steuersignale werden berechnet und jeweils an die im geschlossenen Wirkungsablauf verbundenen Aktoren zur Beeinflussung der Puffervolumen übertragen,
e) Die Aktoren zur Beeinflussung des Puffervolumens rückwirken auf die Sensoren zur Überwachung des Puffervolumens und
f) Schritte b) bis e) werden wiederholt, bis die Produktionsanlage abgeschaltet oder abgefahren ist.

13. Verfahren nach Anspruch 12, wobei im Schritt a) zusätzlich Abschaltbedingungen durch folgende Angabe definiert werden:
∘ ein Maximal- und / oder Minimal-Wert für die Zustandsgröße jedes Puffervolumens wird festgelegt,
∘ ein Maximal- und / oder Minimalwert für die Flussrate jeder Master Unit wird festgelegt.

14. Verfahren nach einem der Ansprüche 12 oder 13, wobei der Wert der Zustandsgröße jedes Puffervolumens im zeitlichen Mittel konstant bleibt.

15. Verfahren nach einem der Ansprüche 12 bis 14, wobei zur Regelung einer Slave Unit eine Fuzzy-Regelung oder eine PID-Regelung, besonders bevorzugt die PID-Regelung verwendet wird.

16. Computer Programm zur Durchführung des Verfahrens nach einem der Ansprüche 1 bis 15.
